# EUROPEAN PATENT SPECIFICATION

(11) **EP 0 464 135 B1**
(45) Date of publication and mention of the grant of the patent: **26.06.1996**
(21) Application number: 90905767.1
(22) Date of filing: 08.03.1990
(51) Int. Cl.: A61K 39/44, A61K 38/43, A61K 38/00, A61K 47/48, C07K 17/06, A61K 31/505

(54) **COMPOSITION FOR REDUCING SIDE EFFECTS OF A DRUG**
ZUSAMMENSETZUNG ZUR HERABSETZUNG DER NEBENWIRKUNGEN EINES ARZNEIMITTELS
COMPOSITION UTILE A REDUIRE LES EFFETS SECONDAIRES D'UN MEDICAMENT

(30) Priority: 13.03.1989 US 322209
(43) Date of publication of application: 08.01.1992
(73) Proprietor: MATSUMURA, Kenneth Naoyuki, Berkeley, CA 94704-2062 (US)
(72) Inventor: MATSUMURA, Kenneth Naoyuki, Berkeley, CA 94704-2062 (US)
(74) Representative: Haibach, Tino, Dr.
(86) International application number: US9001264
(87) International publication number: WO9010460

(56) References cited:
- EP-A- 88 695
- EP-A- 109 861
- EP-A- 0 286 418
- EP-A- 0 330 201
- WO-A-85/00968
- FILE SERVER STN KARLSRUHE,File Biosis, Biological Abstracts no. 82:15341; D. GLOVER et al., no. 86:26092, Biological Abstracts 82:15341; D. GLOVER et al.: "Protects against the hematologoc toxicity of cyclophosphamide a controlled phase II trail" & J. CLIN. ONCOL. 4 (4). 1986. 584-588 #
- HE EMBO JOURNAL, vol. 3, September 1984; MACHY, pp. 1971-1977 #
- iochim. Biophys. Acta, Volume 689, issued 1982, (SHEN) an improved method for covalent attachment of antibody to liposomes pp. 31-37. See pages 32 and 34 (Table1).
- The EMBO J., Volume 3, issued September 1984, (MACHY), Elimination or rescue of cells in culture by specifically targeted liposomes containing methotrexate or formyl-tetrahydrofolate, pp. 1971-1977. See page 1973 and 1974.
- A. GOTH, "Medical Pharmacology", 7th ed. published 1974 by C.V. Mosby Co. (ST. Louis), see pages 648 and 649.
- J. Immunol.Methods 44, 1981, pp. 135 - 151
- CANCER TREATMENT REVIEWS, no. 11, 1984, pages 295-297, Academic Press Inc. (London) Ltd; K. HELLMANN et al.: "Amelioration of antitumor drug toxicity"
- ACTA CLIN. SCAN., suppl. 487, 1978, page 35; B. HUSBERG et al.: "Prolongation of heterotopic rat heart allograft survival by "Methotrexate-folinic acid rescue"

## Description

This invention is in the field of chemotherapy. More specifically, it deals with methods of reducing side effects of chemotherapeutic agents.

Drugs could be made more effective if side effects did not prevent their use in stronger dosages. Side effects are caused by their action not being sufficiently focussed on the body cells or microorganisms needing to be treated. The side effects are specifically caused by misdirected action of said drug on certain body cells which are not the intended target of said drug. For example, if the drug intended for other targets is nevertheless toxic to gastrointestinal cells, then nausea, vomiting, and diarrhea, as well as gastrointestinal bleeding can result. If the drug is toxic to hematopoietic (blood making) cells, anemia, susceptibility to infection (from insufficient number of white corpuscles), and bleeding can result. If the drug is toxic to cells of the skin, hair loss and rash can result.

The reason cancer is so difficult to eradicate from the body is that agents which kill cancer cells also kill normal dividing cells like those of the intestine, hematopoietic bone marrow, and the skin. If normal cells can be protected from the cytotoxic effects of anti-cancer agents, it would become safe to use higher doses of the anti-cancer agents. Since higher doses kill a higher percentage of cancer cells, it would become possible to kill those few cancer cells able to survive the lower dosages currently in use.

The reason viruses are so difficult to eradicate from the body is that agents which kill viruses also kill normal dividing cells. If normal cells can be protected from the cytotoxic effects of anti-viral agents, we would be able to use sufficient dosages of such agents to eradicate life-threatening viruses. For example, azidothymidine (AZT) has been found to be life-prolonging in the treatment of Acquired Immune Deficiency Syndrome (AIDS). According to Backgrounder (published by the National Cancer Institute, Office of Cancer Communications), September 19, 1986, "AZT is a derivative of thymidine, one of the normal components of DNA (genes), which is needed by the host cell and the virus to make chemicals necessary for replication. When AZT enters a cell infected by HTLV-III (the virus that causes AIDS)), the drug floods the cell with false DNA building blocks so the virus cannot make copies of itself. Once this happens, viral infection and replication is halted, thereby protecting target cells." Unfortunately, azidothymidine injected into the blood stream also reaches hematopoietic cells of bone marrow. There, azidothymidine halts replication of reproducing blood cells. The result is anemia and reduced white blood cell count. The NCI Backgrounder (just cited) states, "at high doses, it is likely that bone marrow suppression will be a limiting factor for the drug." If we can protect these bone marrow cells from the harmful action of AZT, we would be able to use a higher dosage of AZT leading to a greater chance of eradicating the AIDS virus.

Other anti-microbial drugs have serious side effects that limit their use. Gentamycin and related drugs like tobramycin and amikacin are, next to penicillin, the most important antibiotics in the treatment of sepsis in debilitated patients (e.g., the post-surgical, the elderly, the immunity-compromised). These drugs, however, also damage kidney cells as well as vestibular and auditory sensory cells and can not often be used in adequate dosages or for sufficient duration. Protecting the kidney, vestibular and auditory sensory cells from the harmful action of these antibiotics can enable more prolonged use of these drugs that can lead to saving of more lives.

Not prepublished prior right EP-A 0 330 201 (NEORX) is generally concerned with the aspect of using antibodies for targeting purposes in the treatment of patients. While the main concern of the reference is with enhancing the activity of cyclotoxic agents on target cells (such as cancer cells to be killed) by suitably targeting either the cytotoxic agent itself, or a 'sensitizing agent' (including WR 2721) by attaching suitable antibody having affinity to the target cells (e.g. cells to be killed) to the substance to be targeted, the reference additionally contemplates that a protecting agent such as WR 2721 may be attached to one or more targeting proteins that bind to nontarget tissues, so that the agent is delivered to nontarget tissues to be protected from cytotoxic agent. As an example it is mentioned that WR 2721 may be targeted to marrow stem cells by conjugating it to an antibody that binds to marrow stem cells.

Biochim. Biophys. ACTA 689, 1982, pp. 31 - 37 is concerned with targeting of drugs (i.e. cytotoxic agents) encapsulated in liposomes, by incorporating antibodies having affinity to the target cells of the drug. More specifically the reference discloses a particular procedure for antibody attachment to the liposomes in the context of such antibody mediated liposome targeting of (cytotoxic) drugs.

EMBO Journal 3/9, 1984, pp. 1971 - 1977 reports in-vitro studies in tissue culture within the context of a specific drugantidote system methotrexate (Mtx) as the drug and tetrahydrofolate (THF) as the associated antidote. In this context the reference discloses - in an in-vitro situation in tissue culture - the protection of one population of cells (by THF) while another population of cells in the same culture is killed by the drug (Mtx). The THF-antidote is enclosed or incorporated in a liposome carrier and the carrier is targeted by antibody. Similarly, EP-A-O 109 861 (BIO-RESPONSE INC.) exclusively relates to an in-vitro-tissue culture environment, and in this context suggests rescuing a sub-population of desired cells (in tissue-culture) from a noxious environment (such as formed by methotrexate drug) by a protective agent such as folinic acid encapsulated in a suitable carrier such as liposomes having attached thereto an 'anti-product' (e.g. an antibody) for targeting to the desired cells to be rescued. Quite similarly, J. Immunol. Methods 44, 1981, pp. 135 - 151 pertains exclusively to an in-vitro tissue culture situation, and in this context discloses an immuno-selection technique utilizing antibody specificity to deliver a protecting enzyme (catalase) to cells subsequently placed in a noxious environment (H₂O₂); catalase-anti-catalase complexes are formed and are bound to the surface of the target cell (i.e. the cell to be protected from H₂O₂) by a bridging antibody with specificity for both the complex and cell surface antigen-specific antibody.

Dialogue Information Services, Inc., File 159 relating to 'the delivery of radiosensitizers to hypoxic tumor-cells using monoclonal antibodies' exemplifies the technique, known per se, of drug targeting by antibodies covalently attached to the drug; again, this reference is mainly concerned with in-vitro tissue culture work.

EP-A-0 286 418 (WEINSTEIN) discloses a particular type of liposome to be used as a carrier for encapsulating a particular drug, namely an anti-retroviral agent having toxicity against the HIV retro-virus causing aids, and passim mentions that said carrier liposome carrying said anti-retroviral agent can be antibody-targeted to the noxious species.

The present invention purports to providing an effective means of avoiding or minimizing, within a living organism such as a patient undergoing treatment, the undesired and potentially damaging side effects of a drug administered to said organism, by co-ordinated administration of a targeted antidote, in a manner and under conditions that will efficiently work in the complex immunological system of a living organism.

To this purpose, in accordance with a basic embodiment of the present invention there is provided an antidote composition for in-vivo administration to an organism in correlation with and correspondence to in-vivo administration of a drug to said organism, where there exists for said drug an antidote capable of reducing the cellular effect of said drug, whereby to reduce that drug's side effects caused by undesired effects of said drug upon body cells in said organism which are not the intended target of said drug, said antidote composition when administered to said organism providing for preferential delivery of said antidote to said body cells to be protected from the undesired effects of said drug, wherein said antidote composition comprises said antidote having attached thereto noncomplement fixing antibody with affinity for said body cells (to be protected from said drug).

In accordance with a further basic embodiment of the present invention, provision is made for an antidote composition for in-vivo administration to an organism in correlation and correspondence to in-vivo administration of a drug to said organism where there exists for said drug an antidote capable of reducing the cellular effect of said drug, whereby to reduce that drug's side effects caused by undesired effects of said drug upon body cells in said organism which are not the intended target of said drug, said antidote composition when administered to said organism providing for preferential delivery of said antidote to said body cells to be protected from the undesired effects of said drug, wherein said antidote composition comprises said antidote placed within a non-negatively charged uni-lamellar liposome of diameter size of less than approximately 600 A, said antidote-carrying liposome having attached thereto antibodies with affinity for said body cells (to be protected from said drug).

By thus utilizing antibody of a particular type, namely of the non-complement fixing type, for antidote targeting, and/ or by using a particular type of liposome, namely non-negatively charged liposome of small size, as carrier for that antidote and targeting said carrier, the system of the present invention provides for a particularly efficient and straight-forward focusing of drug effect and reducing side effects of a drug by protecting body cells not meant to be affected by said drug from the unwanted effects of said drug. The present invention provides for an efficient means to preferentially deliver to the body cells needing said protection an antidote for said drug when said drug is used, said targeted delivery being accomplished by binding antibodies with affinity for said body cells to said antidote or to carriers or liposomes holding said antidote. Described in a different way, when a first chemical is an antidote for a second chemical, my invention is the use of the first chemical bound to antibodies from the class comprising antibodies with affinity to bone marrow cells, antibodies with affinity to gastrointestinal cells, antibodies with affinity to oropharyngeal cells, antibodies with affinity to respiratory cells, and antibodies with affinity to basal keratinocyte cells, when the second drug is being used to treat other targets in an organism.

By the term "drug", I mean chemotherapeutic agents such as those listed in The Pharmacologic Basis of Therapeutics (A.G. Gilman, L.S. Goodman, A. Gilman, editors. MacMillan Publishing Co. New York. 1980 or later editions). For this invention, the term "drug" includes ionizing radiation when used in treatment of diseases since the radioactive high energy particles act as drugs.

By the term "antidote", I mean a chemical that counteracts the cellular effect of a specific drug. For example, folinic acid is an antidote for anti-cancer drug methotrexate, thymidine is an antidote for anti-cancer drugs 5-fluorouracil (5-FU) & floxuridine (FUdR), and for AIDS drug AZT, uridine is an antidote for azaribine, and deoxycytidine is an antidote for cytosine arabinoside. Folinic acid is a weak antidote for vinblastine and vincristine, but glutamic and aspartic acids are antidotes for vinblastine.

There is a certain logic about determining what chemical would serve as an antidote for any given drug:
1) If the drug acts by being a non-functional analog of a natural metabolite (for example, AZT or 5-FU), one antidote for such a drug is the natural (functional) metabolite for which this drug is the analog (for AZT or 5-FU, thymidine).
2) If the drug acts by reversibly blocking an enzyme critically needed for the production of a specific metabolite (for example, the enzyme dihydrofolate reductase is blocked by methotrexate in the production of thymidine), one antidote for such a drug is the specific metabolite whose production is being blocked by the drug (for example, in the case of methotrexate, thymidine is also an antidote, according to the logic just delineated).
3) If natural resistance to a given drug is found to develop in cells, one can learn how cells become resistant and find an antidote at the same time. For example, cells often become more resistant to the action of a drug by increasing the synthesis of an enzyme that can help degrade the drug once the drug enters the cell. Said enzyme is an antidote for the drug. As a corollary to this rule, an enzyme that can function otherwise harmlessly in the intracellular milieu which degrades the drug can be an antidote. Some cells are resistant to the action of bleomycin because of the higher intracellular concentration of bleomycin hydrolase. Resistance to cytosine arabinoside has also been attributed to cytidine deaminase. Resistance to important aminoglycoside antibiotics has been attributed to acetylases and aminoglycoside-inactivating enzymes. Xanthine oxidase found plentifully in the liver detoxifies 6-mercaptopurine and azathioprine. These enzymes, when delivered into the cytoplasm of cells needing protection, are antidotes for the drugs they detoxify.
4) For ionizing radiation as a drug, one can find antidotes among chemicals known to be "radioprotectors", such as WR-2721 and WR-1065 (Walter Reed Army Institute of Research, Washington, D.C.).
5) A new approach I am still exploring is to deliver into the cellular cytoplasm an exogenous excess of the target structures to which the drug binds (cisplatin to deoxyribonucleic acid), intercalates with (doxorubicin, daunorubicin with deoxyribonucleic acid), or directly alters (alkylators with DNA and components like guanine). In this case, fragments of DNA (e.g., guanine-base rich DNA fragments, artificially synthesized) would be the antidote. Once inside the cytoplasm, DNA fragments could react with entering cytotoxic drug molecules rendering them harmless to the "real" functioning DNA of the cell.
6) Some drugs act via generation of free-radicals inside cells (for example, doxorubicin). In such cases, free-radical scavengers like alpha-tocopherol and coenzyme Q can serve as antidotes. Doxorubicin's use is limited by its cardiotoxicity which appears to be particularly caused by free-radical generation. Aforementioned scavengers can serve as antidotes to the heart muscle cells.

When seeking antidotes, one can use known empirical evidence or conduct in vivo or in vitro tests. For example, one can determine if a substance qualifies as an "antidote" for the purposes of this invention as follows: one introduces the test "antidote" into a tissue culture of body cells needing protection. Then the drug for which the antidote is being tested is introduced into the tissue culture. One assesses the effect that the test "antidote" had on the action of the drug on the cells in tissue culture so that one can determine the acceptability of the test "antidote". For some antidotes, like proteins and enzymes, one may need to provide means for the test antidote to get inside the cultured body cells (for example, by placing the enzymes inside liposomes (discussed below) and allowing the enzymes to enter the cytoplasm when the membranes of the liposomes and the cell fuse.

There are many ways one can "preferentially deliver (the antidote) to the body cells needing ... protection." One can covalently bind the antidote to antibodies having affinity to the body cells needing protection. The state of the art continues to advance in methodology but I refer to Hurwitz, E., Cancer Res. 35: 1175-, 1975 who describes a successful linkage via periodate oxidation of the drug to be bound and reaction of the oxidized drug with the antibody followed by borohydride reduction of the reagents. Particularly useful technique is that developed by Cytogen Corporation (Princeton, New Jersey, USA - Rodwell, J. D. et al. Proc. Nat. Acad. Sci. 83: 2632 -2636, 1986) which uses the oligosaccharides found in the constant region of the heavy chains of an antibody. Chemical or enzymic oxidation of oligosaccharides to aldehydes generates groups that react with compounds containing such functional groups as amines, hydrazines, hydrazides, and thiosemicarbazides. This site-specific modification yields relatively uniform antibody conjugates that have unimpaired antigen binding characteristics. These "linker" conjugates are also "conditionally stable," i.e., the linkers release drugs in response to a so-called "second signal." Drugs linked to the antibodies attach to the target cells and a second signal, such as a proteolytic enzyme, breaks up the conjugates, releasing the drug into the extracellular space surrounding the target cells. In addition to enzymes (such as complement, plasmin, and elastase), linkers can also be made sensitive to pH changes, or radioisotopes, for example. The use of the "second signal" is not always necessary since a cell will take into the interior drugs that attach to its outer membrane.

Prior to the work of Cytogen Corporation, generally, it was felt difficult to directly bind drugs onto the antibody without causing the loss of function of the drug or the antibody. Therefore, many found it preferable to bind the drug first to a carrier and the carrier in turn bound to the antibody. In this manner, it is possible to bind more molecules of a drug to the antibody without causing the loss of function of the antibody. A successful example is that of M. Garnett, M.J. Embleton, E. Jacobs & R.W. Baldwin (Int. J. of Cancer : 661-670, 1983) who use albumin as a carrier. Reisfeld and his group lately has been using conjugation technique employing an acid-sensitive linker, cis-aconitic anhydride (Proc. Nat. Acad. Sci. 85: 1189 - 1193, 1988). Another interesting, though less disirable, technique is the use of polyglutamic acid as an intermediate carrier, as described by G. F. Rowland (Nature 255: 487-, 1975).

In a preferred embodiment and especially when using antidotes which are enzymes, the antidotes are "bound" to antibodies by first placing them within liposomes. Then antibodies are covalently attached to the walls of liposomes in a manner that preserves antibody's ability to recognize and associate with the antigens on the target cell surface. Such a technique will be described later.

Liposomes are spherules formed when phospholipids are allowed to swell in aqueous compartments. Within the lipid or aqueous phase of liposomes, lipid or water-soluble substances, respectively, can be entrapped. Several of physical properties of liposomes can be varied at will: size (radius) can be adjusted from about 12 nm for unilamellar liposomes to up to several microns for the multilamellar versions, and a negative or positive surface charge can be imposed by the incorporation of charged amphiles (Gregoriadis, G., Methods in Enzymology 44: 698 - . 1976). Control of permeability to entrapped substances, and of stability is also feasible by the addition of a sterol or other lipids into the liposomal structure (Gregoriadis. G., reference just cited).

These liposomes can be made to home in on target cells by attaching antibodies (Barbet, J., Machy, P., & L. Leserman, J. Supramolecular Structure and Cellular Biochemistry 16: 243 - 258. 1981 (Cellular Recognition, p. 237 - 252); Leserman, L., Barbet, J., & F. Kourilsky, Nature 288: 602 -604. 1980; Machy, P., Pierres, M., Barbet, J., & L. Leserman, J. Immunology 129: 2098 - 2102. 1982; Machy, P., & L. Leserman, EMBO J. 3(9): 1971 - 1977. 1984; Konno, H. et al. Cancer Res. 47: 4471 - 4477, 1987).

For the purpose of the present invention, the liposome is unilamellar, less than 60 nm in diameter and non-negatively about charged. Many methods are well-known in the art for making suitable liposomes (Juliano. R. L., Stamp, D., Biochem. Biophys. Res. Comm. 63 (3): 651 - 658, 1975; also Ann. New York Acad. Sci. 308: 411 - 432, 1978, also Canad. J. Physio. Pharmacy 57 (5): 535 - 539, 1979, and Biochem. Pharmacol. 27: 21 - 27, 1978; Kimelberg, H.K., Cancer Res. 36: 2949 - 2957, 1976; work of Barbet, J., Machy, P., & L. Leserman, previously cited; Gregoriadis, G. Nature 265: 407 - 411, 1977; Gregoriadis, G., New England J. Med. 295 (3): 704 - 710, 1976 and 295 (14): 765 - 770, 1976). Plentiful information is available in the research literature that describes how to vary the size, charge and content of the liposomes (Gregoriadis, G., Leathwood, P.D., & Ryman, B.E., Fed. Europ. Biochem. Soc. Letters 14: 95 -99, 1971; Gregoriadis, G., Fed. Europ. Biochem. Soc. Trans. 2: 117 -119, 1974; Gregoriadis, G. & Ryman, B.E., Europ. J. Biochem. 24: 485 - 491, 1972; Magee, E. E., Miller, O.V., Nature 235: 339 - 340, 1972; Kobayashi, T., Gann 66: 719 - 720, 1975; Gregoriadis, G. Biochem. Soc. Trans. 2: 117, 1974; Gregoriadis, G. Fed. Europ. Bioch. Soc. Letters 36 (3): 292-1973; Gregoriadis, G. & E.D. Neerunjun Biochem. Biophys. Res. Comm. 65: 537 -544, 1975).

Suitable antibodies for delivering the liposomes to target cells can be made by standard immunological methods, including that described by G. Gregoriadis (Biochem. Biophysic. Res. Comm. 65: 537 - 544. 1975). Monoclonal antibody production methods, however, are the best way to provide large amounts of purified antibodies (Kohler, G. & C. Milstein, Nature 256: 495 - 497. 1975; Barbet, J., Machy, P., & L. Leserman, cited above). The antibodies used in this invention should be non-complement-fixing. There are now many suitable commercially available antibodies (e.g., MRX OX1 mouse anti-rat leukocyte common antigen; OKT-10 mouse anti-human bone marrow cells). For the bone marrow stem cells and precursor cells of blood corpuscles and platelets, one can use antibodies comparable to those active against T200 glycoprotein of the mouse bone marrow cells. For the gastrointestinal cells, it would be preferable to use IgG₁ monoclonal antibodies against colon and small intestinal crypt and stem cells' laterobasal membrane. Other cells of the alimentary system needing protection includes oropharyngeal, esophageal, and gastric cells. For the skin cells, it would be preferable to use monoclonal IgG₁ antibodies against basal keratinocytes (and hair follicle keratinocytes). While some normal dividing cells divide less frequently and may not need protection when anti-mitotic cytoxic drugs are used, such cells may need protection under certain circumstances of drug use, such as in prolonged use or ultra-high dose. The previous sentence refers to cells including respiratory epithelial cells, urinary epithelial cells, and hepatocytes. Some drugs have specific organ toxicity (amikacin antibiotics to renal cells and otic cells; doxorubicin to cardiac muscle cells) and in such cases antibodies with affinity to the affected organs would be used.

The dosage of the antidote delivered to cells needing protection must be adequate to provide said protection. For example, in using folinic acid, one must deliver to the cell being protected at least one mole of folinic acid for each mole of methotrexate entering said cell. There may be advantages in injecting liposomes directly into the arteries feeding the cells needing protection (e.g., coeliac and superior mesenteric arteries feeding gastrointestinal cells).

The antidote should be introduced into the actively circulating body fluid (blood) bathing the cells needing protection before the cytotoxic drug is introduced into the same medium so as to allow the antidote time to reach its target cells. While antidote like folinic acid has been found to attenuate the toxic effects of methotrexate even when administered 3 hours following methotrexate, attenuation is greater sooner one administers the antidote. In the case of this invention, the antidote preferably would be administered from two to twelve hours before the cytotoxic drug because it takes time for the antidote to be delivered to the cells needing protection because the antibody-guided delivery is relatively slow. Of course, one can hasten the delivery by increasing the amount of antibody-bound-antidote, but economic consideration could limit that approach.

One can make in advance of need antidote-carrier-antibody complexes (including antidote inside liposome bound to antibody). For example, any drug which has been found to be an antidote to another drug can be complexed with antibody to bone marrow stem cells, with antibody to colonic cells, with antibody to basal keratinocyte cells, etc. and each type of complex can be kept on cryogenic storage shelves for later use.

The following are examples of how to practice the invention. They are presented merely as an illustration and should not be construed to mean that the scope of the invention is limiting to the examples or even that the examples necessarily represent the best modes of operation.

Example 1. Unilamellar liposomes are made containing folinic acid (sodium salt) using egg phosphatidyl choline, cholesterol, and dipalmitoyl phosphatidyl ethanolamine 3-(2-pyridyldithio) propionate in molar ratio of 64:35:1 and to the liposomes are bound antibodies with affinity to bone marrow precursors of the white blood corpuscles) using a method adapted from Barbet (J. Supramol. Structure & Cell Biochem. previously cited). These liposomes are injected intravenously several hours prior to the administration of methotrexate for the treatment of cancer. Bone marrow toxicity of methotrexate is considerably reduced. It is particularly advantageous to simultaneously use 1) liposomes binding antibodies with affinity to gastrointestinal, esophageal, and oropharyngeal cells, 2) liposomes binding antibodies with affinity to cells of the skin, and 3) liposomes binding antibodies with affinity to the bone marrow stem cells, all the liposomes containing folinic acid or thymidine. Methotrexate can then be safely administered at a dosage easily two to three times that usually tolerated. In the case of liposomes containing thymidine, 5-FU and methotrexate together may be used. When using thymidine, the drug 5-fluorouridine may be used.

Example 2. Unilamellar liposomes are made containing thymidine and to the liposomes are bound antibodies with affinity to bone marrow stem cells, as in Example 1. These liposomes are injected intravenously several hours prior to the administration of azidothymidine for the treatment of AIDS. Bone marrow toxicity of AZT is considerably reduced. AZT is used at over 150% the usual dosage.

Example 3. As in Example 1 except liposomes incorporating cytidine deaminase instead of folinic acid and the drug is cytosine arabinoside instead of methotrexate.

Example 4. As in Example 1 except liposome incorporating bleomycin hydrolase instead of folinic acid and the drug is bleomycin instead of methotrexate.

Example 5. As in Example 1 except liposomes incorporating uridine instead of folinic acid and the drug is azaribine instead of methotrexate.

Example 6. As in Example 1 except liposome incorporating deoxycytidine instead of folinic acid and the drug is cytosine arabinoside instead of methotrexate.

Example 7. Instead of using liposomes as in above examples, the antidote is bound to antibodies using acid-sensitive linker, cis-aconitic anhydride. Otherwise, as in Examples 1, 2, 5, and 6.

Other examples include substituting the above drugs and their antidotes with other drug-antidote pairs mentioned in this specification.

A further example: my invention can also be used to create disease models in organisms by allowing a cytotoxic drug to kill or make sick certain body cells while protecting other body cells that can be affected by the cytotoxic agent. For example, one can used 5-FU to damage colonic mucosal cells to simulate colitis while protecting small intestine, stomach, esophageal, oropharyngeal, bone marrow, and basal keratinocyte cells from the cytotoxic action of 5-FU by the preferential delivery to the latter cells of thymidine using the methods described.

## Claims

1. An antidote composition for in-vivo administration to an organism in correlation with and correspondence to in-vivo administration of a drug to said organism, where there exists for said drug an antidote capable of reducing the cellular effect of said drug, whereby to reduce that drug's side effects caused by undesired effects of said drug upon body cells in said organism which are not the intended target of said drug, said antidote composition when administered to said organism providing for preferential delivery of said antidote to said body cells to be protected from the undesired effects of said drug,
wherein said antidote composition comprises said antidote having attached thereto a non-complement fixing antibody with affinity for said body cells (to be protected from said drug).

2. The antidote composition of claim 1 wherein said antidote is any from the group consisting of folinic acid, thymidine, deoxycytidine, uridine, glutamic acid, and aspartic acid.

3. An antidote composition for in-vivo administration to an organism in correlation with and correspondence to in-vivo administration of a drug to said organism where there exists for said drug an antidote capable of reducing the cellular effect of said drug, whereby to reduce that drug's side effects caused by undesired effects of said drug upon body cells in said organism which are not the intended target of said drug, said antidote composition when administered to said organism providing for preferential delivery of said antidote to said body cells to be protected from the undesired effects of said drug,
wherein said antidote composition comprises said antidote placed within a non-negatively charged unilamellar liposome of diameter size of less than approximately 60 nm, said antidote-carrying liposome having attached thereto non-complement fixing antibodies with affinity for said body cells (to be protected from said drug).

4. The antidote composition of claim 3 wherein said antidote is any from the group consisting of thymidine, folinic acid, deoxycytidine, uridine, glutamic acid, aspartic acid, bleomycin hydrolase, xanthine oxidase, and fragments of deoxyribonucleic acid.

5. Unilamellar, non-negatively charged liposome of diameter size of less than approximately 60 nm which encloses an antidote in its aqueous compartment and to which are bound non-complement fixing antibodies from the class comprising: antibodies with affinity to bone marrow progenitor cells, antibodies with affinity to gastrointestinal cells, antibodies with affinity to urinary epithelial cells, antibodies with affinity to respiratory epithelial cells, antibodies with affinity to oropharyngeal cells, antibodies with affinity to basal keratinocyte cells of the skin.

6. The antidote composition of claim 1 or claim 3, wherein said antibody is any from the group consisting of antibodies with affinity for T200 glycoprotein of bone marrow cells, IgG₁ antibodies against intestinal crypt and stem cells' lateralbasal membrane, and IgG₁ antibodies against basal and hair follicle keratinocytes.

7. The unilamellar liposome of claim 5 wherein said antibodies with affinity to gastrointestinal cells have affinity to intestinal crypt and stem cells' lateral-basal membrane.

## Patentansprüche

1. Antidot-Zusammensetzung zur in-vivo Verabreichung an einen Organismus in Korrelation mit und in Entsprechung zur in-vivo Verabreichung eines Arzneimittels an den genannten Organismus, soweit für das genannte Arzneimittel ein Antidot existiert, das die Zellularwirkung des genannten Arzneimittels zu reduzieren vermag, um so die Nebeneffekte des Arzneimittels zu verringern, die durch unerwünschte Wirkungen des genannten Arzneimittels auf Körperzellen in dem genannten Organismus bewirkt werden, welche nicht das beabsichtigte Target bzw. Ziel des genannten Arzneimittels sind, wobei die genannte Antidot-Zusammensetzung bei Verabreichung an den genannten Organismus eine bevorzugte Zufuhr des genannten Antidots an die gegen die unerwünschten Wirkungen des genannten Arzneimittels zu schützenden Körperzellen ergibt, wobei die genannte Antidot-Zusammensetzung das genannte Antidot aufweist, an welchem ein nicht-komplementbindender Antikörper mit Affinität für die genannten (gegen das genannte Arzneimittel zu schützenden) Körperzellen angebracht ist.

2. Antidot-Zusammensetzung nach Anspruch 1, bei welcher das genannte Antidot eines aus der aus Folinsäure, Thymidin, Deoxycytidin, Uridin, Glutaminsäure und Asparaginsäure bestehenden Gruppe ist.

3. Antidot-Zusammensetzung zur in-vivo Verabreichung an einen Organismus in Korrelation mit und in Entsprechung zur in-vivo Verabreichung eines Arzneimittels an den genannten Organismus, soweit für das genannte Arzneimittel ein Antidot existiert, das die Zellularwirkung des genannten Arzneimittels zu reduzieren vermag, um so die Nebeneffekte des Arzneimittels zu verringern, die durch unerwünschte Wirkungen des genannten Arzneimittels auf Körperzellen in dem genannten Organismus bewirkt werden, welche nicht das beabsichtigte Target bzw. Ziel des genannten Arzneimittels sind, wobei die genannte Antidot-Zusammensetzung bei Verabreichung an den genannten Organismus eine bevorzugte Zufuhr des genannten Antidots an die gegen die unerwünschten Wirkungen des genannten Arzneimittels zu schützenden Körperzellen ergibt, und wobei die genannte Antidotzusammensetzung das genannte Antidot eingelagert in einem nichtnegativgeladenen, unilamellaren Liposom mit einer Durchmesserabmessung von weniger als etwa 60 nm enthält, wobei an dem genannten antidottragenden Liposom nichtkomplementbindende Antikörper mit Affinität für die genannten (gegen das genannte Arzneimittel zu schützenden) Körperzellen angebracht sind.

4. Antidot-Zusammensetzung nach Anspruch 3, bei welcher das Antidot eines aus der aus Thymidin, Folinsäure, Deoxycytidin, Uridin, Glutaminsäure, Asparaginsäure, Bleomycinhydrolase, Xanthinoxidase und Fragmenten von Deoxyribonucleinsäure bestehenden Gruppe ist.

5. Unilamellares, nicht-negativgeladenes Liposom der Durchmesserabmessung von weniger als etwa 60 nm, welches in seinem wäßrigen Abteil ein Antidot einschließt und an welches nicht-komplementbindende Antikörper aus der Klasse gebunden sind, welche umfaßt: Antikörper mit Affinität zu Knochenmarks-Progenitorzellen, Antikörper mit Affinität zu Gastrointestinalzellen, Antikörper mit Affinität zu Harnepithelialzellen, Antikörper mit Affinität zu Respirationsepithelialzellen, Antikörper mit Affinität zu Oropharyngealzellen, Antikörper mit Affinität zu Basal-Keratinozytenzellen der Haut.

6. Antidot-Zusammensetzung nach Anspruch 1 oder Anspruch 3, bei welcher der genannte Antikörper einer aus der Gruppe ist, welche aus Antikörpern mit Affinität für T200 Glycoprotein von Knochenmarkszellen, Antikörpern gegen Intestinalkrypt und Lateralbasalmembran von Stammzellen, sowie IgG₁ Antikörper gegen Basal- und Haarfollikel-Keratinocyten besteht.

7. Unilamellares Liposom nach Anspruch 5, bei welchem die genannten Antikörper mit Affinität zu Gastrointestinalzellen Affinität zu Intestinalkrypt und Lateralbasalmembran von Stammzellen besitzen.

## Revendications

1. Composition antidote pour l'administration in vivo à un organisme en corrélation avec et en correspondance à l'administration in vivo d'un médicament à cet organisme, un antidote existant pour ce médicament et qui est capable de réduire l'effet cellulaire de ce médicament, permettant de réduire les effets secondaires de ce médicament provoqués par les effets indésirables de ce médicament sur les cellules corporelles de cet organisme et qui ne sont pas la cible recherchée de ce médicament, la composition antidote lorsqu'elle est administrée à l'organisme assurant, pour l'amenée préférentielle de l'antidote aux cellules corporelles, la protection vis-à-vis des effets indésirables de ce médicament,
la composition antidote comprenant l'antidote avec, fixé sur celui-ci, un anticorps de fixation non complémentaire ayant une affinité pour les cellules corporelles (qu'il s'agit de protéger vis-à-vis de ce médicament).

2. Composition antidote selon la revendication 1, dans laquelle l'antidote est un quelconque antidote choisi dans le groupe constitué par l'acide folinique, la thymidine, la désoxycytidine, l'uridine, l'acide glutamique et l'acide aspartique.

3. Composition antidote pour l'administration in vivo à un organisme en corrélation et en correspondance avec l'administration in vivo d'un médicament à cet organisme, un antidote existant pour ce médicament capable de réduire l'effet cellulaire de ce médicament, permettant de réduire les effets secondaires du médicament provoqués par les effets indésirables de ce médicament sur les cellules corporelles dans l'organisme et qui ne sont pas la cible envisagée de ce médicament, la composition antidote lorsqu'elle est administrée à l'organisme assurant, pour une amenée préférentielle du l'antidote aux cellules corporelles, la protection vis-à-vis des effets indésirables de ce médicament,
la composition antidote comprenant l'antidote placé à l'intérieur d'un liposome unilamellaire chargé non négativement, d'un diamètre inférieur à environ 60 nm, le liposome porteur d'antidote comportant fixés sur celui-ci des anticorps de fixation non complémentaires ayant une affinité pour les cellules corporelles (qu'il s'agit de protéger vis-à-vis de ce médicament).

4. Composition antidote selon la revendication 3, dans laquelle l'antidote est un quelconque antidote choisi dans le groupe constitué par la thymidine, l'acide folinique, la désoxycytidine, l'uridine, l'acide glutamique, l'acide aspartique, l'hydrolase de bléomycine, l'oxydase de xanthine et des fragments de l'acide désoxyribonucléique.

5. Liposome unilamellaire chargé non négativement d'un diamètre inférieur à environ 60 nm qui renferme un antidote dans son compartiment aqueux et eut lequel sont liés des anticorps de fixation non complémentaires de la classe comprenant : les anticorps ayant une affinité pour les cellules progénitrices de la moelle osseuse, des anticorps ayant une affinité pour les cellules gastro-intestinales, des anticorps ayant une affinité pour les cellules épithéliales urinaires, des anticorps ayant une affinité pour les cellules épithéliales respiratoires, les anticorps ayant une affinité pour les cellules oropharyngiennes, les anticorps ayant une affinité pour les cellules kératinocytes basales de la peau.

6. Composition antidote selon la revendication 1 ou 3, dans laquelle l'anticorps est un quelconque anticorps choisi dans le groupe constitué par les anticorps ayant une affinité pour la glycoprotéine T200 des cellules de la moelle osseuse, des anticorps IgG₁ vis-à-vis de la membrane latérobasale des cellules souches et des cryptocellules intestinales et des anticorps IgG₁ vis-à-vis des kératinocytes folliculaires pileuses et basales.

7. Liposome unilamellaire selon la revendication 5, dans lequel les anticorps ayant une affinité pour des cellules gastro-intestinales ont une affinité pour la membrane latérobasale des cellules souches et des cryptocellules intestinales.
